Europäisches Patentamt

European Patent Office

Office européen des brevets

(11) Publication number: 0 476 205 A1

# EUROPEAN PATENT APPLICATION

(21) Application number: 90313718.0

(22) Date of filing: 14.12.90

(51) Int. Cl.5: **A61K 31/485**, A61K 9/02, A61K 47/36, A61K 47/38, A61K 47/40, A61K 47/48

(30) Priority: 21.09.90 JP 253134/90

(43) Date of publication of application: 25.03.92 Bulletin 92/13

(84) Designated Contracting States: AT BE CH DE DK ES FR GB GR IT LI LU NL SE

(71) Applicant: TORII & CO., LTD. 4-1, Nihonbashi-Honcho-3-chome Chuo-ku Tokyo(JP)

(72) Inventor: Uekama, Kaneto 1716-80, Nagaminemachi Kumamoto-shi(JP)

(74) Representative: Silveston, Judith et al ABEL & IMRAY Northumberland House 303-306 High Holborn London, WC1V 7LH(GB)

(54) Sustained release morphine preparation for rectal administration.

(57) A sustained release hollow type suppository for rectal administration comprising a mixture of morphine, α-cyclodextrin and a thickener.

FIG. I

MORPHINE CONCENTRATION IN PLASMA (ng/ml)

○ : MORPHINE HYDROCHLORIDE + LACTOSE

● : MORPHINE HYDROCHLORIDE + α-CYCLODEXTRIN + XANTHAN GUM

TIME AFTER SUPPOSITROY ADMINISTRATION (HOUR)

The present invention relates to a sustained release preparation for rectal administration comprising a mixture of morphine, α-cyclodextrin and a thickener.

In more particular, the invention relates to a hollow type suppository containing morphine which shows a high bioavailability in rectal administration and a sustained effective concentration in blood.

In order to enable end-stage cancer patients to be relieved from severe pains and to complete a life more worthy of man, the World Health Organization (WHO) proposed in 1984 the use of opium-group analgesics of strong action (World Health Organization: "Cancer pain relief", WHO, Geneva, 1986). Morphine preparations currently in use include, besides previous peroral preparations (powders and tablets) and injections, sustained release tablets of morphine sulfate (Kiso to Rinsho (Basic and Clinical Medicine), 21, 279-292, No. 15, 1987). Peroral preparations have a drawback in that, since they are susceptible to first pass effect caused by the intestinal organizations and the liver, they show a low bioavailability and as much as two thirds of the morphine administered is excreted before entering the circulatory system. Further, when peroral administration of morphine is difficult for such reasons as the site of cancer development, pathological conditions etc., morphine suppositories become necessary.

Many studies have been made on morphine suppositories. The advantage of morphine suppository administration as compared with peroral administration lies in that the rectal administration causes a less extent of gastrointestinal troubles, is less susceptible to first pass effect and hence shows a higher bioavailability.

Known morphine suppositories include conventional morphine hydrochloride suppositories prepared at pharmacist's offices, morphine suppositories excellent in absorbability through the rectum (Uekama Kaneto et al., Sixth Meeting of Japan DDS Society, July 14, 1990, held at Nagasaki city), and suppositories with rapid-release (rapid-acting) property. In the treatment of cancerous pains, to lighten the burdens of patients and the persons engaged in medical services, suppositories which have a sustained analgesic effect and require less number of times of administration per day are also useful. One of such sustained release morphine suppositories known previously is that obtained by molding a mixture of morphine, an acidic polysaccharide, and an oily or fatty base (Japanese Patent Application Kokai (Laid-open), H-2-36,123 (1990)). However, no satisfactory morphine suppository of sustained release has been obtained yet.

The present inventors have made extensive study to solve the above problems and resultantly succeeded to create a suppository for rectal administration markedly improved in bioavailability and sustainability of concentration in blood, by admixing morphine, α-cyclodextrin and a thickening agent, as hereinafter defined, and filling the resulting mixture into a hollow suppository. Thus the present invention has been achieved.

The morphine may be in the form of morphine hydrochloride or morphine sulphate.

A thickening agent is a substance that has the property of increasing the viscosity of water when dissolved or dispersed in water. Thickening agents are well known, see for example, Martindale "The Extra Pharmacopoeia" Ed. J.E.F. Reynolds, The Pharmaceutical Press, London, 29th Edition, 1989.

Examples of thickening agents that may be used according to the present invention include those listed below under the heading "Thickening Agents":

Thickening Agents

Acacia (gum arabic); agar; alginic acid; sodium alginate; aluminium magnesium silicate; arabogalactan; bentonite; carbomer (carboxypolymethylene); carmellose (carboxymethylcellulose); carrageenan; cellacephate (cellulose acetate phthalate); dispersible cellulose; microcrystalline cellulose; powdered cellulose; ceratonia (locust bean gum); dextrates; ethylcellulose; β-1,3-glucan; hydroxyethylcellulose; hydroxymethylcellulose; hydroxypropylcellulose; hypromellose (hydroxypropylmethylcellulose); hypromellose phthalate (hydroxypropyl - methylcellulose phthalate); linseed; magnesium silicate; methylcellulose; pectin; povidone (polyvinyl pyrrolidone); propylene carbonate; purified siliceous earth; silicon dioxide; colloidal silicon dioxide; slippery elm; sodium starch glycollate; tragacanth (gum tragacanth) and xanthan gum.

Locust bean gum, arabogalactan, β-1,3-glucan, hydroxypropylcellulose and xanthan gum are particularly mentioned as thickening agents for use in the sustained release suppositories of the present invention.

The suppository of the present invention can be prepared by known methods (cf., for example, Japanese Patent Application Kokai (Laid-open) No. 59-21,613, and Nobuaki Matsumoto et al., Shindan to Chiryo (Diagnosis and Therapy), 77 (4), 895-898, 1989) in the following manner.

α-Cyclodextrin, for example, natural α-cyclodextrin, morphine and a thickening agent are admixed, the proportions of the components being, for example, 5-100 parts by weight, preferably 20-50 parts by weight of α-cyclodextrin to 1 part by weight of morphine and, for example, 5-100 parts by weight, preferably 20-50 parts by weight of the thickening agent to 1 part by weight of morphine.

The resulting mixture is filled into a hollow suppository prepared with a conventional suppository base, whereby a sustained release suppository containing morphine, for example, morphine hydrochloride according to the present invention is obtained.

Though the reason is not definitely clear why the sustained morphine concentration in blood and the enhanced bioavailability are attained by mixing α-cyclodextrin and a thickening agent with morphine according to the present invention, it is conceivably because the morphine concentration at the absorption site of the rectum is maintained at a high level for a long time with the aid of the thickening agent and the absorption of morphine into the living body is improved by the action of α-cyclodextrin on the rectal mucous membrane or by the surface activity thereof.

The present invention provides the use of a thickening agent for the manufacture of a sustained release filled hollow type suppository containing a mixture of morphine and α-cyclodextrin for the relief of pain.

The present invention also provides the use of a thickening agent to enhance the bioavailability of morphine from a filled hollow type suppository containing morphine and α-cyclodextrin and/or to sustain the concentration in the blood of morphine from such a suppository.

The present invention further provides a thickening agent for or in admixture with morphine and α-cyclodextrin as the filling for the manufacture of a sustained release hollow type suppository for the relief of pain.

The present invention will be described further in detail below with reference to Example and Test Example.

Example

Preparation of hollow type suppository containing a morphine hydrochloride-α-cyclodextrin-xanthan gum mixture

In an agate mortar were placed 20 mg of morphine hydrochloride, 400 mg of α-cyclodextrin and 400 mg of xanthan gum, then 5 ml of water was added and the whole was milled; further, 5 ml of water was added thereto, and the resulting mixture was milled for 1 hour in all. The milled product was dried under reduced pressure at room temperature for 1 day, and the resulting solid mixture was ground and sieved through a No. 100 sieve. As a control, a milled product was also prepared by using lactose in place of α-cyclodextrin and xanthan gum.

The hollow type suppository was prepared by using the Witepsol® H-15 base and with a 3 g suppository mold. The hollow part had an inside diameter of 5 mm and a space volume of about 0.5 cm$^3$. The intended suppository was prepared by filling in a hollow suppository 205 mg each (containing 5 mg of morphine hydrochloride) of the mixture of morphine hydrochloride with α-cyclodextrin and xanthan gum or the mixture (control) of morphine hydrochloride with lactose obtained above.

Test Example

The present suppository and the control suppository prepared in the Example were respectively administered into the rectum of a native Japanese white rabbit weighing about 2 kg and the morphine concentration in the plasma of the rabbit was determined.

The rabbit was fasted for about 48 hours before administration, but given water ad libitum.

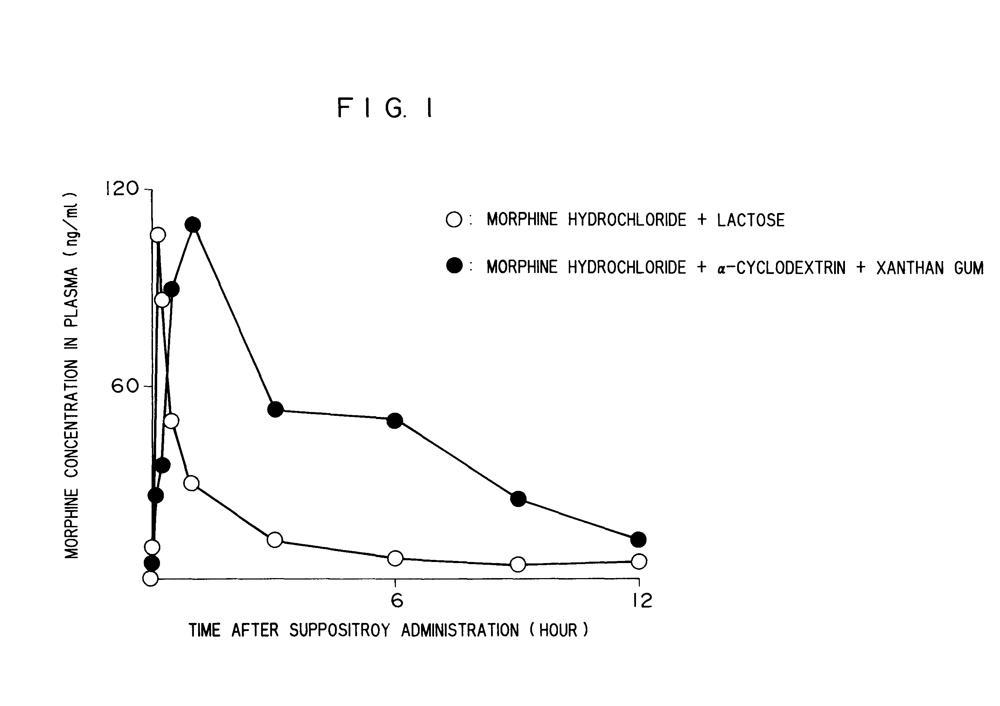

Each of the suppositories prepared in the Example was inserted into the rectum of the rabbit and the anus was pinched with a clip to prevent the leakage of the suppository ingredients. Blood samples were collected from the ear vein 0 minute, 5 minutes, 15 minutes, 30 minutes, 1 hour, 3 hours, 6 hours, 9 hours and 12 hours after the suppository administration, and the concentrations of morphine in the plasma were determined according to the method of Katagiri et al. (Journal of Pharmacy and Pharmacology, 40, 879-881 (1988)). The results are shown in Fig. 1. In the Figure, the solid line with circles represents the curve of the concentration in plasma after administration of the hollow type suppository of the control (containing 5 mg of morphine hydrochloride) filled with a mixture of morphine hydrochloride and lactose, while the dotted line with black circles represents that after administration of the present hollow type suppository filled with a mixture of morphine hydrochloride, α-cyclodextrin and xanthan gum (weight ratio = 1 : 20 : 20; containing 5 mg of morphine hydrochloride).

As is apparent from the Figure, the present suppository takes a longer time to reach the maximum concentration in blood ($T_{max}$) as compared with the control suppository; on the other hand, it gives a maximum morphine concentration ($C_{max}$) of the same level and an area under the blood concentration-time curve (AUC) of 3.7 times as large. These results demonstrate a sustained morphine concentration in blood.

BRIEF DESCRIPTION OF THE DRAWING

Fig. 1 is a graph showing the concentration in plasma exhibited when the present suppository (indicated by black circles) and the control suppository (indicated by circles) are administered to rabbits.

In the Figs. ○-○ refers to the suppository comprising morphine alone and ●-● to the suppository comprising a combination of morphine-α-cyclodextrin complex and xanthan gum.

**Claims**

1. A sustained release hollow type suppository for rectal administration containing a mixture of morphine, α-cyclodextrin and a thickening agent as hereinbefore defined.

2. A suppository as claimed in claim 1, wherein the thickening agent is at least one member selected from the group consisting of xanthan gum, locust bean gum, arabogalactan, α-1,3-glucan and hydroxypropylcellulose.

3. A suppository as claimed in claim 1 or claim 2, wherein the morphine is morphine hydrochloride or morphine sulphate.

4. A suppository as claimed in any one of claims 1 to 3, wherein the weight ratio of morphine to α-cyclodextrin is in the range of from 1 : 5 to 1 : 100.

5. A suppository as claimed in any one of claims 1 to 3, wherein the weight ratio of morphine to α-cyclodextrin is in the range of from 1 : 20 to 1 : 50.

6. A suppository as claimed in any one of claims 1 to 5, wherein the weight ratio of morphine to the thickening agent is in the range of from 1 : 5 to 1 : 100.

7. A suppository as claimed in any one of claims 1 to 5, wherein the weight ratio of morphine to the thickening agent is in the range of from 1 : 20 to 1 : 50.

8. Use of a thickening agent for the manufacture of a sustained release filled hollow type suppository containing a mixture of morphine and α-cyclodextrin for the relief of pain.

9. Use of a thickening agent to promote the bioavailability of morphine from a filled hollow type suppository containing morphine and α-cyclodextrin and/or to sustain the concentration in the blood of morphine from such a suppository.

10. A thickening agent for or in admixture with morphine and α-cyclodextrin as the filling for the manufacture of a sustained release hollow type suppository for the relief of pain.

**Claims for the following Contracting States: ES, GR**

1. A process for the manufacture of a filled sustained release hollow type suppository for rectal administration comprising mixing morphine, α-cyclodextrin and a thickening agent as hereinbefore defined, and filling the resulting mixture into a hollow type suppository.

2. A process as claimed in claim 1, wherein the thickening agent is at least one member selected from the group consisting of xanthan gum, locust bean gum, arabogalactan, α-1,3-glucan and hydroxypropyocellulose.

3. A process as claimed in claim 1 or claim 2, wherein the morphine is morphine hydrochloride or morphine sulphate.

4. A process as claimed in any one of claims 1 to 3, wherein the weight ratio of morphine to α-cyclodextrin is in the range of from 1 : 5 to 1 : 100.

5. A process as claimed in any one of claims 1 to 3, wherein the weight ratio of morphine to α-cyclodextrin is in the range of from 1 :20 to 1 : 50.

6. A process as claimed in any one of claims 1 to 5, wherein the weight ratio of morphine to the thickening agent is in the range of from 1 : 5 to 1 : 100.

7. A process as claimed in any one of claims 1 to 5, wherein the weight ratio of morphine to the thickening agent is in the range of from 1 : 20 to 1 : 50.

8. Use of a thickening agent for the manufacture of a sustained release filled hollow type suppository containing a mixture of morphine and α-cyclodextrin for the relief of pain.

9. Use of a thickening agent to promote the bioavailability of morphine from a filled hollow type suppository containing morphine or a salt thereof and α-cyclodextrin and/or to sustain the concentration in the blood of morphine from such a suppository.

10. A thickening agent for or in admixture with morphine and α-cyclodextrin as the filling for the manufacture of a sustained release hollow type suppository for the relief of pain.

# F I G. I

MORPHINE CONCENTRATION IN PLASMA ( ng/ml )

120

60

○ : MORPHINE HYDROCHLORIDE + LACTOSE

● : MORPHINE HYDROCHLORIDE + α-CYCLODEXTRIN + XANTHAN GUM

6          12

TIME AFTER SUPPOSITROY ADMINISTRATION ( HOUR )

EP 0 476 205 A1

## European Patent Office

# EUROPEAN SEARCH REPORT

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | STN INTERNATIONAL INFORMATION SERVICES DATA BASE: CHEMICAL ABSTRACTS, vol. 113, no. 12, accession no. 103421u, Columbus, Ohio, US; <br> & JP-A-02 036 123 (DAITO KOEKI CO., LTD) 06-02-1990 <br> * Abstract * <br> – – – | 1-3,8-10 | A 61 K 31/485 <br> A 61 K 9/02 <br> A 61 K 47/36 <br> A 61 K 47/38 <br> A 61 K 47/40 <br> A 61 K 47/48 |
| D,A | EP-A-0 094 157 (TAKEDA) <br> * Claims 1,6-7; page 16, lines 13-36; page 17, lines 23-35 * <br> – – – – – | 1-3,8-10 | |
| | | | **TECHNICAL FIELDS SEARCHED (Int. Cl.5)** <br><br> A 61 K |

**The present search report has been drawn up for all claims**

| Place of search | Date of completion of search | Examiner |
|---|---|---|
| The Hague | 13 November 91 | SCARPONI U. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document